# EUROPÄISCHE PATENTANMELDUNG

(11) **EP 2 280 002 A1**
(43) Veröffentlichungstag der Anmeldung: **02.02.2011**
(21) Anmeldenummer: 09009823.7
(22) Anmeldetag: 29.07.2009
(51) Int. Cl.: C07D 213/12

(54) **Verfahren zur selektiven Herstellung von 3-Methylpyridin (3-Picolin) aus Acrolein und einem oder mehreren in Wasser gelösten Ammoniumsalzen**

(71) Anmelder: Lonza Ltd., 4052 Basel (CH)
(72) Erfinder: Die Erfindernennung liegt noch nicht vor

(57) **Zusammenfassung**

Die vorliegende Erfindung betrifft ein Verfahren zur selektiven Herstellung von 3-Methylpyridin, **dadurch gekennzeichnet, dass** Acrolein und ein oder mehrere in Wasser gelöste(s) Ammoniumsalz(e) unter hohen Drücken und Temperaturen von 200-400 °C kontinuierlich umgesetzt werden.

## Beschreibung

### Verfahren zur selektiven Herstellung von 3-Methylpyridin (3-Picolin) aus Acrolein und einem oder mehreren in Wasser gelösten Ammoniumsalzen.

Die vorliegende Erfindung betrifft ein Verfahren zur selektiven und kontinuierlichen Herstellung von 3-Methylpyridin aus Acrolein.

Ein wesentlicher Anteil an 3-Methylpyridin wird als Ausgangsstoff zur Herstellung von Insektiziden, z. B. Chlorpyrifos, oder Pharmazeutika genutzt. Auch kann es bei der Produktion von Nahrungsergänzungsmitteln wie Nicotinsäure oder Nicotinsäureamid, sowie Herbiziden wie Fluazifopbutyl eingesetzt werden [Shi2005].

Nach dem Stand der Technik kann Acrolein u. a. aus der Dehydratisierung von Glycerol, das als "Abfallprodukt" in größeren Mengen bei der Biodiesel-Produktion anfällt, in nah- und überkritischem Wasser unter Zugabe von Schwefelsäure [Wat2007] oder Salzen [Ott2006] hergestellt werden. Die direkte Zugabe von Ammoniakwasser oder einer unter den Reaktionsbedingungen Ammoniak liefernden Substanz in dieses Ausgangsgemisch führt nicht zu den erwünschten Ausbeuten an 3-Methylpyridin. Das gewonnene Acrolein wird daher anschließend in einem weiteren Schritt zu 3-Methylpyridin umgesetzt.

Es ist bekannt, dass bei der Umsetzung von Acrolein mit Ammoniak in der Gasphase in Gegenwart von Katalysatoren 3-Methylpyridin entsteht. Als Katalysatoren dienen vornehmlich Substanzen auf der Grundlage von Oxiden und Silikaten des Aluminiums. Es werden Fluorokieselsäure oder Fluoroborsäure enthaltende Aluminiumsilikate, die durch Erhitzen auf 450 °C vorbehandelt sind (DT-OS 1 917 037) oder zeolitische Molekularsiebe mit Gehalten an Lanthan eingesetzt (DT-OS 2 023 158). Nachteilig sind die geringen Raum-Zeit-Ausbeuten dieser Verfahren.

In erheblichem Umfang entsteht neben 3-Methylpyridin Pyridin bei Einsatz von Katalysatoren, die aus bei Temperaturen von 550 bis 1200 °C mit Sauerstoff vorbehandelten Verbindungen aus den Elementen Al, F und O und mindestens einem Element der zweiten, dritten und vierten Gruppe des Periodensystems (DE-OS 2 151 417) oder aus mindestens zwei Elementen der zweiten, vierten fünften und sechsten Gruppe des Periodensystems (DE-OS 2 224 160) oder mindestens ein Element der zweiten Gruppe des Periodensystems (DE-OS 2 239 801) bestehen.

In geringen Ausbeuten erhält man 3-Methylpyridin auch bei der Herstellung aus Acrolein, Propionaldehyd und Ammoniak mit Katalysatoren bestehend aus Aluminiumoxid, Siliziumoxid und gegebenenfalls Zusätzen von Oxiden weiterer Elemente (FR-PS 1 273 826).

Durch Einsatz hochdisperser Aluminiumsilikate kann die Ausbeute an 3-Methylpyridin auf etwa 60 % gesteigert werden (DE-OS 2 703 070).

Kristalline Zeolithe, mit einem Silizium zu Aluminium-Verhältnis von mindestens 12 und einem Constraint Index von 1 bis 12, z. B. ZSM5, werden in US 4 220 783 für die Umsetzung von C2-C4 Aldehyden, C3-C5 Ketonen oder Mischungen besagter Aldehyde und/oder Ketone mit Ammoniak in Gegenwart von Methanol oder Wasser eingesetzt. Geringe Lebensdauer des Katalysators sowie Ausbeuten an Pyridin und 3-Picolin sind nachteilig für dieses Verfahren. Durch Einsatz synthetischer, poröser und kristalliner Materialien, z.B. Zeolith MCM-22 oder MCM-49, als Katalysator, können die Ausbeuten an Pyridin und 3-Alkylypridinen erhöht werden (US 5 395 940). Hierbei werden als Reaktanden Formaldehyd, C2-C4 Aldehyde, C3-C5 Ketone oder Mischungen besagter Aldehyde und/oder Ketone, sowie Ammoniak und Wasserstoff eingesetzt.

Aus DE 3 634 259 ist bekannt, dass ein Gemisch aus Acrolein und Alkanalen mit Ammoniak in Gegenwart eines Zeolithen des Pentasiltyps selektiv zu 3-Methylpyridin ohne größeren Zwangsanfall an Pyridin umgesetzt wird. Eine Ausbeute von 91 % an 3-Methylpyridin kann über 6 Stunden in einem Rohrreaktor durch Umsetzung von Acrolein und Propionaldehyd mit Ammoniak in einem dreifachen stöchiometrischen Überschuss erhalten werden. Die Reaktionstemperatur liegt bei 400 °C und eine Regeneration des Katalysators ist notwendig.

Ebenso ist bekannt, Acrolein mit Ammoniumsalzen in einer diskontinuierlichen Verfahrensweise, einem sauren Reaktionsmilieu, z. B. Propionsäure, und bei Temperaturen von 15-150 °C umzusetzen (GB-PS 1 240 928). Die Ausbeuten an 3-Methylpyridin sind mit etwa 33 % relativ gering.

Die Herstellung von 3-Metylpyridin aus Acrolein oder einem Gemisch von Acrolein und Formaldehyd oder einem Gemisch von Acrolein, Formaldehyd und Acetaldehyd in flüssiger, wässriger Phase bei Temperaturen von 180-280 °C in einem geschlossenen Gefäß in Gegenwart von Ammoniak und/oder Ammoniumsalzen, wie z. B. Diammonium-hydrogenphosphat, ist aus EP 0 075 727 sowie aus Grayson, J.I und Dinkel, R. "An Improved Liquid-Phase Synthesis of Simple Alkylpyridines" Helvetica Chimica Acta, Vol. 67 (1984), 2100-2110, bekannt. Nach dem Verfahren wird 3-Methylpyridin in Ausbeuten unter 60 % erhalten und die Bildung von Pyridin weitgehend unterdrückt. Nachteilig sind die langen Zugabezeiten des Aldehyds in die Reaktionslösung von 20-90 Minuten.

Das zu lösende technische Problem bestand nun darin, das produzierte Acrolein in einer zweiten Stufe kontinuierlich in hohen Ausbeuten ohne Einsatz von Katalysatoren und mit kurzen Verweilzeiten zu 3-Methylpyridin umzusetzen. Dieses Problem wird durch das erfindungsgemäße Verfahren, welches dadurch gekennzeichnet ist, dass Acrolein und ein oder mehrere in Wasser gelöste(s) Ammoniumsalz(e) unter hohen Drücken und Temperaturen von 200-400 °C kontinuierlich umgesetzt werden, gelöst.

Das erfindungemäße Verfahren läuft bevorzugt in einem pH-Bereich von 4-8, besonders bevorzugt von 4-6 ab.

Es ist erfindungsgemäß besonders bevorzugt, die Umsetzung im sauren Reaktionsmilieu durchzuführen, wodurch die Bildung von Metallhydroxiden und/oder Polymerisationsreaktionen des Acroleins verhindert werden kann.

Besonders bevorzugt sind dabei anorganische Ammoniumsalze, insbesondere Ammoniumsulfat, Ammoniumacetat und Ammoniumdihydrogenphosphat.

Dabei liefern die Ammoniumsalze unter den erfindungsgemäßen Reaktionsbedingungen Ammoniak, welches mit Acrolein unter Bildung eines Heterocyclen umgesetzt wird. Es stellte sich überraschenderweise heraus, dass fast ausschließlich 3-Methylpyridin gebildet wird, jedoch nicht Pyridin und/oder weitere Pyridin-Derivate, welche durch anschließende aufwendige Aufarbeitungsschritte vom erwünschten Produkt getrennt werden müssten.

Das erfindungsgemäße Verfahren erreicht eine maximale Ausbeute an 3-Picolin von 35-60 % bezogen auf die eingesetzten Ausgangsverbindungen.

Es kann erfindungsgemäss bevorzugt sein, das Acrolein aus Glycerin mittels im Stand der Technik bekannter Verfahren zu gewinnen.

Als Hauptnebenprodukte des erfindungsgemäßen Verfahrens fallen Acetaldehyd und Formaldehyd an. Dieses Gemisch kann z.B. als Ausgangssubstanz zur Rückgewinnung von Acrolein und/oder zur Herstellung von 3-Methylpyridin eingesetzt werden.

Das erfindungsgemäße Verfahren kann sowohl direkt mit dem Acrolein enthaltenden Reaktionsgemisch des Acrolein-Syntheseschrittes als auch mit vorher gereinigtem Acrolein durchgeführt werden.

Abhängig von der Dichte des Mediums werden erfindungsgemäß bevorzugt Verweilzeiten von 5-400 s, besonders bevorzugt 150-300 s eingestellt.

Die Umsetzungen erfolgen erfindungsgemäß bevorzugt bei maximal 400 °C und 40 MPa.

Das erfindungsgemäße Verfahren kann in standardmäßigen Hochdruckanlagen durchgeführt werden. Bevorzugt wird hierbei eine Anlage mit einem Strömungsrohrreaktor aus Inconel625 und einem Reaktorvolumen von 4-50 mL. Die Ausgangsmischungen werden über zwei vorgeheizte, separate Stränge mit maximal 35 ml min⁻¹ in den Reaktor gefördert.

Die Erfindung wird durch die nachfolgenden, nicht-limitierenden Beispiele erläutert.

### Beispiel 1

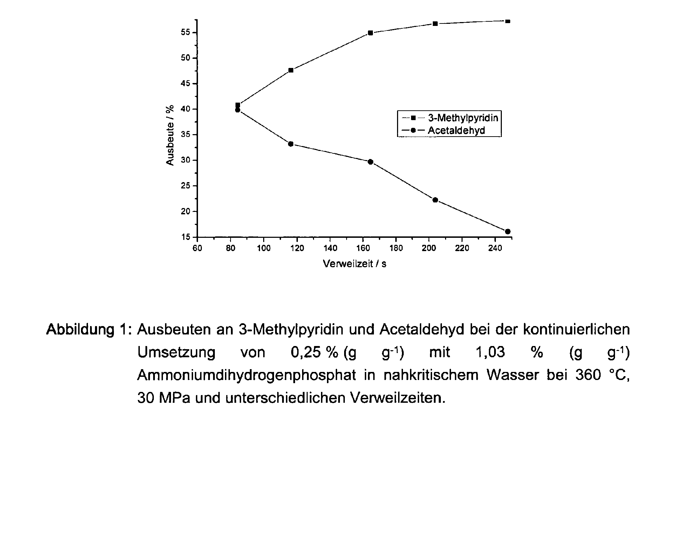

Eine wässrige Lösung mit 0,75 % (g g⁻¹) Acrolein und 3,07 % (g g⁻¹) Ammoniumdihydrogenphosphat, was einem Molverhältnis von Acrolein zu Ammoniumdihydrogenphosphat von 1:2 entspricht, wird in einer zweisträngigen Hochdruckanlage bei 30 MPa umgesetzt. Das flüssige Gemisch wird zunächst in einer Vorheizung auf 170 °C erwärmt und anschließend mit der doppelten Menge an heißem Wasser gemischt, so dass am Reaktoreingang eines Rohrreaktor aus Inconel625 und Volumens von 49,5 mL, die Reaktionstemperatur von 360 °C eingestellt wird und nahkritische Bedingungen des Wassers herrschen.. Die Reaktionslösung wird anschließend in einem Wärmetauscher auf Raumtemperatur abgekühlt und auf Atmosphärendruck entspannt. In einem Phasenseperator werden bei 2 °C die flüssigen Komponenten von den gasförmigen getrennt. Die flüssige Phase wird gesammelt und die Anteile der nachweisbaren Komponenten gaschromatographisch bestimmt. Zur quantitativen Bestimmung des Pyridins und dessen Derivaten wird die saure Probenlösung mit Ammoniakwasser auf einen pH-Wert von 7-8 eingestellt. Als interner Standard wird 3,5-Dimethylpyridin verwendet, das in vorhergehenden Untersuchungen nicht nachgewiesen werden konnte. Zur Bestimmung des Acetaldehyds und des Acroleins wird die Probe lediglich mit 1-Butanol als interner Standard versetzt. Die ermittelten Ausbeuten von 3-Methylpyridin und Acetaldehyd unter den beschriebenen Bedingungen sind in Abbildung 1 dargestellt. Die maximale Ausbeute an 3-Methylpyridin beträgt 57 % bei einer Verweilzeit von 248 s. Pyridin fällt mit einer Ausbeute von maximal 2 % in unbedeutenden Mengen an. Andere Pyridin-Derivate werden nur in Spuren gebildet.

### Beispiel 2

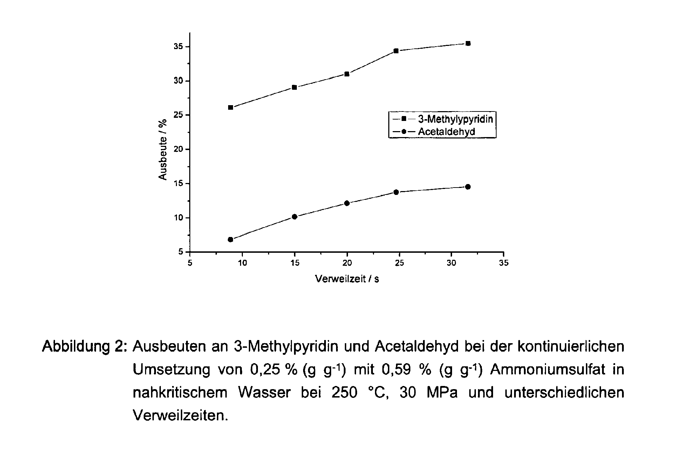

Die Umsetzung erfolgt mit Acrolein und Ammoniumsulfat in einem Molverhältnis von 1:1. Eine wässrige Lösung mit 0,75 % (g g⁻¹) Acrolein wird zunächst in einer Vorheizung auf 50 °C erwärmt und anschließend mit der doppelten Menge einer vorgewärmten wässrigen Lösung mit 0,89 % (g g⁻¹) Ammoniumsulfat gemischt, so dass eine Reaktionstemperatur von 250 °C am Reaktoreingang eines Strömungsrohrreaktors aus Inconel625 mit einem Reaktorvolumen von 4,4 mL eingestellt wird. Je nach Volumenfluss und Dichte des Reaktionsmediums werden Verweilzeiten von 5-35 s eingestellt. Die Ergebnisse sind in Abbildung 2 dargestellt. Die maximale Ausbeute an 3-Methylpyridin beträgt 36 % bei einer Verweilzeit von 32 s. Pyridin fällt mit einer Ausbeute von maximal 1 % an, wobei andere Pyridin-Derivate wiederum nur in Spuren gebildet werden.

### Beispiel 3

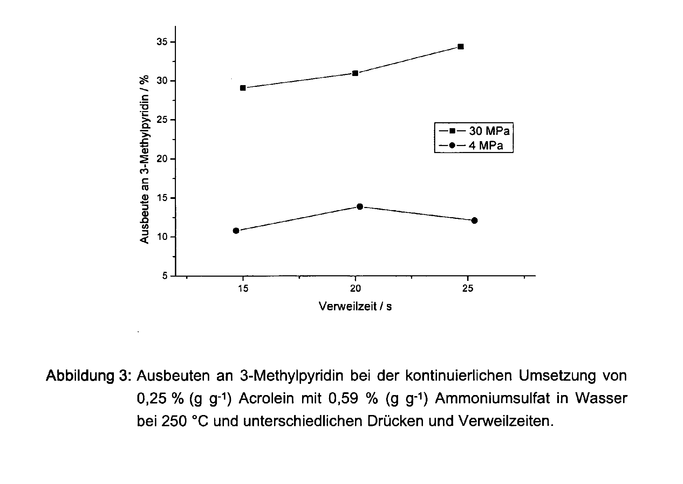

Die Umsetzung erfolgt entsprechend Beispiel 2. Es wird eine Druckeinstellung vorgenommen, so dass der Betriebsdruck leicht über dem Dampfdruck der Reaktionslösung liegt. In Abbildung 3 sind die Ausbeuten an 3-Methylpyridin bei 4 MPa und 30 MPa gegenübergestellt.

Die maximale Ausbeute an 3-Methylpyridin liegt bei einem niedrigen Reaktionsdruck mit 14 % deutlich unter den erhaltenen Werten bei Anwendung höherer Drücke.

### Beispiel 4

Eine wässrige Lösung mit 1,00 % (g g⁻¹) Glycerol und 0,05 % (g g⁻¹) Zinksulfat wird entsprechend Beispiel 1 bei 25 MPa umgesetzt. Das flüssige Gemisch wird zunächst in einer Vorheizung auf 230 °C erwärmt und anschließend mit der doppelten Menge an heißem Wasser gemischt, so dass am Reaktoreingang die Reaktionstemperatur von 360 °C eingestellt wird und nahkritische Bedingungen des Wassers herrschen. Die Verweilzeit im Reaktor beträgt 140 s. Der Nachweis aller flüssigen Komponenten erfolgt gaschromatographisch mit N-Methyl-2-pyrrolidon als interner Standard. Bei einem Umsatz von Glycerol von 73 % werden Acrolein und Acetaldehyd mit Ausbeuten von 20 % bzw. 28 % erhalten.

In einem zweiten Reaktionsschritt wird die aus dem ersten Reaktionsschritt erhaltene Reaktionslösung mit einer Acrolein-Konzentration von 0,12 % (g g⁻¹) Acrolein mit der äquimolaren Menge an Ammoniumsulfat versetzt und bei 360 °C, 30 MPa und einer Verweilzeit von 160 s umgesetzt. Hierbei wird das Eduktgemisch unverdünnt in den Reaktor eingeleitet. Man erhält 3-Methylpyridin mit einer Ausbeute von 43 %. Die Gesamtausbeute von 3-Methylpyridin bezüglich Glycerol beträgt 8 %.

### Literatur

[Ott2006] L. Ott, M. Bicker, H. Vogel: Catalytic dehydration of glycerol in sub- and supercritical water: a new chemical process for acrolein production, Green Chemistry, 2006, 8, 214-220.
[Shi2005] S. Shimizu, N. Watanabe, T. Kataoka, T. Shoji, N. Abe, S. Morishita, H. Ichimura: Pyridine and Pyridine Derivatives, Ullmann's Encyclopedia of Industrial Chemistry, 7. Aufl., Wileylnterscience, Online Release, 2005**.**
[Wat2007] M. Watanabe, T. lida, Y. Aizawa, T. M. Aida, H. Inomata: Acrolein synthesis from glycerol in hot-compressed water, Bioresource Techno/ogy, 2007, 98, 1285-1290.

## Patentansprüche

1. Verfahren zur selektiven Herstellung von 3-Methylpyridin, **dadurch gekennzeichnet, dass** Acrolein und ein oder mehrere in Wasser gelöste(s) Ammoniumsalz(e) unter hohen Drücken und Temperaturen von 200-400 °C kontinuierlich umgesetzt werden.

2. Verfahren gemäß Anspruch 1, **dadurch gekennzeichnet, dass** Ammoniumsulfat, Ammoniumacetat und/oder Ammoniumdihydrogenphosphat eingesetzt werden.

3. Verfahren gemäß mindestens einem der Ansprüche 1-2, **dadurch gekennzeichnet, dass** das Acrolein und das Ammoniumsalz in einem Molverhältnis von 1:0,125 bis 1:2 eingesetzt werden.

4. Verfahren gemäß mindestens einem der Ansprüche 1-3, wobei der Druck so eingestellt wird, dass das Reaktionsgemisch flüssig-einphasig vorliegt.

5. Verfahren gemäß mindestens einem der Ansprüche 1-4, **dadurch gekennzeichnet, dass** die Umsetzung bei Drücken von 20-40 MPa erfolgt.

6. Verfahren gemäß mindestens einem der Ansprüche 1-5, **dadurch gekennzeichnet, dass** die Kontakt- bzw. Verweilzeit 5-400 s, bevorzugt 150-300s beträgt.

7. Verfahren gemäß mindestens einem der Ansprüche 1-6, wobei der pH-Wert der wässrigen Lösung in einem Bereich von 4-8 liegt.

8. Verfahren gemäß mindestens einem der Ansprüche 1-7, wobei die Ausbeute an 3-Picolin 35-60 % bezogen auf die eingesetzten Ausgangsverbindungen beträgt.

9. Verfahren gemäß mindestens einem der Ansprüche 1-8, wobei als Nebenprodukte Acetaldehyd und Formaldehyd erhalten und optional dem Verfahren wieder zugeführt werden.

10. Verfahren gemäß Anspruch 9, wobei die Ausbeute an Acetaldehyd zwischen 35-50 % bezogen auf die eingesetzten Ausgangsverbindungen beträgt.

11. Verfahren gemäß mindestens einem der Ansprüche 1-10, wobei das Acrolein aus Glycerin gewonnen wird.
